(19) Europäisches Patentamt
European Patent Office
Office européen des brevets

(11) **EP 3 842 062 A1**

# (12) EUROPEAN PATENT APPLICATION
published in accordance with Art. 153(4) EPC

(43) Date of publication:
**30.06.2021 Bulletin 2021/26**

(21) Application number: **19851847.4**

(22) Date of filing: **15.08.2019**

(51) Int Cl.:
**A61K 38/42** (2006.01)   **C07K 14/805** (2006.01)

(86) International application number:
**PCT/CL2019/050073**

(87) International publication number:
**WO 2020/037441 (27.02.2020 Gazette 2020/09)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(30) Priority: **20.08.2018 CL 20182378**

(71) Applicant: **Universidad Austral de Chile**
**Valdivia, 5110566 (CL)**

(72) Inventors:
• **LOPEZ SIERRA, Mauricio**
**Valdivia, 5110566 (CL)**

• **TURNER BUSCHMANN, Alice**
**Valdivia, 5110566 (CL)**
• **RYBERTT SALINAS, Soledad**
**Valdivia, 5110566 (CL)**
• **GARCES CATALAN, Juan Luis**
**Valdivia, 5110566 (CL)**
• **VALENZUELA OLEA, Guillermo**
**Valdivia, 5110566 (CL)**
• **RUMINOT BASCUR, Marcos**
**Valdivia, 5110566 (CL)**

(74) Representative: **Herrero & Asociados, S.L.**
**Cedaceros, 1**
**28014 Madrid (ES)**

(54) **MEDICINAL PRODUCT USEFUL AS A BLOOD SUBSTITUTE FOR SIMULTANEOUSLY TREATING ACUTE ANAEMIA DUE TO BLOOD LOSS AND BACTERIAL INFECTION IN MAMMALS**

(57)    The present invention relates to use of the extracellular haemoglobin isolate purified from an annelid native to Chile, preferably from the species *Abarenicola pusilla,* to prepare a medicinal product useful as a blood substitute for simultaneously treating acute anaemia due to blood loss and bacterial infection in mammals. The present invention also relates to a pharmaceutical composition for treating acute anaemia due to blood loss and bacterial infection in mammals, comprising an effective concentration of an extracellular haemoglobin isolate from an annelid native to Chile, preferably from the species *Abarenicola pusilla,* and a pharmaceutically acceptable excipient.

EP 3 842 062 A1

**Description**

FIELD OF APPLICATION OF THE INVENTION

The present invention relates to a simultaneous treatment of anaemia and bacterial infection in mammals by means of the administration of an adequate amount of extracellular hemoglobin isolated from a sea worm native to Chile.

DESCRIPTION OF KNOWN MATTER

Hemoglobins are proteins that circulate in the blood of all vertebrates and some invertebrates, and are characterized by transporting oxygen from the respiratory organs to all the tissues of the body, and removing carbon dioxide from the same for its elimination. Vertebrate hemoglobin is made up of a heterotetramer, with a heme group containing an iron molecule attached to each monomer (Hardison R. A brief history of hemoglobins: Plant, animal, protist, and bacteria. Proc. Natl. Acad. Sci USA 1996 Vol 93: 5675-79).

This vital function can be affected in cases of blood loss such as serious trauma caused by accidents, surgical interventions, and obstetric complications, anaemia due to malnutrition, or diseases such as thalassemia or sickle cell disease, among others. When there is a loss of blood, there is a decrease in blood volume, blood pressure decreases and can result in an eventual death of the organism. Estimates indicate that the limit for blood loss is around 30% to 40% of the total volume present in the body.

In order to compensate blood loss, it is necessary to resort to blood transfusions. Each country must ensure that its blood reserves are sufficient to supply the constant need of patients. For this, donors are recruited, which allow to obtain resources that can be stored in Blood Banks. Unfortunately, blood can only be kept for a limited period of time, after which it must be discarded. For this reason, a constant flow of donors is required to maintain the reserves and thus ensure the availability of blood at any time.

According to data from the World Health Organization (WHO), the minimum necessary to meet the most basic needs of a country is having 10 donors per 1,000 inhabitants. In transition and developing countries, this average value is 7.5 and 2.3 donors per 1,000 inhabitants, respectively. In addition, it should be considered that donated blood must be free of any type of contamination, that is, conducting at least detection tests for HIV, hepatitis B and C viruses, and syphilis and emerging pathogens (zika, yellow fever, etc.) for its acceptance.

Therefore, there is a deficit in blood reserves mainly in developing or transition countries that must be covered in some manner. One way to face this deficit is by replacing the blood with any material that can supply the composition and functionality of the blood.

To date, different alternatives for blood transfusion have been considered, among which are substitutes based on perfluorocarbon compounds, which are capable of carrying and releasing a high volume of oxygen to the tissues, but when administered intravenously can produce pulmonary embolism, failure of blood pressure, and eventual death of the animals (Castro C., Briceno JC. Perfluorocarbon-Based Oxygen Carriers: Review of Products and Trials Artif Organs. 2010. Vol 34: 622-634). Another alternative is hemoglobin-based oxygen carriers, which can come from different origins (human, bovine, recombinant, invertebrates). Human hemoglobin is obtained after the lysis of red cells, which loses its tetrameric structure and divides into two dimers, reducing its molecular weight. Being smaller, the renal system removes this hemoglobin quickly, causing nephrotoxicity (Winslow R. Johns Hopkins University Press. 1992). In addition, being of human origin, its availability is limited, and it carries a significant risk of pathogen transmission. For this reason, bovine hemoglobin is a low cost, readily available alternative, but it has been observed that it presents a high risk of prion transmission, it is difficult to purify, and as a consequence of the presence of cellular debris in the preparations, it generates antibody production after transfusion (Hemoglobin-based solutions. Network for the Advancement of Patient Blood Management Haemostasis and Thrombosis (http://www.nataonline.com/np/421/hemoglobin-based-solutions).

Recombinant hemoglobin has been produced in *E. coli,* and has been modified to solve the problem of dimer dissociation and improve oxygen affinity, however it still presents purification problems, since patients who underwent a recombinant hemoglobin transfusion suffered typical symptoms of endotoxin toxicity (Shoemaker SA, Gerber MJ, Evans GL, Archer-Paik LE, Scoggin CH. Initial clinical experience with a rationally designed, genetically engineered recombinant human hemoglobin. Artif Cells Blood Substit Immobil Biotechnol. 1994. Vol 22: 457-465). This technology has not yet been optimized for production on an industrial scale. The United States Food and Drug Administration, FDA, has controlled clinical tests of hemoglobins from different companies in healthy volunteers, patients in surgery and trauma, who resulted with serious heart, liver, gastrointestinal problems, among others (Weiskopf R, Silverman T. Balancing potential risks and benefits of hemoglobin-based oxygen carriers Transfusion. 2013. Vol 53: 2327-2333). For these reasons, it is necessary to search for a blood substitute that meets the ideal conditions of good availability, low cost, non-toxic, efficient in terms of carrying and delivering oxygen, compatible with all blood types, easy to store, and that has a long shelf life.

Various invertebrates, including annelids and nematodes, have been described in the literature as containing extracellular hemoglobin with high molecular weight. In the document Garlick R. L., Terwilliger R. C. Structure and oxygen equilibrium of hemoglobin and myoglobin from the pacific lugworm Abarenicola pacifica. Comp. Biochem. Physiol. 1977. Vol 57B: 177-184, a hemoglobin from *Abarenicola pacifica* with high molecular weight is described. On the other hand, in the document Rousselot M, Delpy E, Drieu La Rochelle C, Lagente V, Pirow R, Rees JF, Hagege A, Le Guen D, Hourdez S, Zal F. Arenicola marina extracellular hemoglobin: a new promising blood substitute. Biotechnol J. 2006 Vol 1: 333-345, a new extracellular protein from *Arenicola marina* is described, which is proposed as a potential blood substitute. Assays in mice indicated that this protein is not potentially toxic. The affinity for oxygen of this hemoglobin was evaluated according to the parameter P50, defined as the partial pressure of oxygen necessary to saturate half of the binding sites. Therefore, the higher the P50 value, the weaker the binding of oxygen to hemoglobin. In the physiological conditions typical of *A. marina,* the affinity of its hemoglobin is high (P50 = 2.6 mmHg), but in human physiological conditions, this affinity is reduced (P50 = 7 mmHg). This research group has filed a series of patent applications regarding this protein and its use as a blood replacement treatment and other applications (US Patent No. 7,776,526, US Patent Application No. 12/110,936, US Patent Application No. 10/296,982, US Patent Application No. 20120040453, US Patent No. 7,999,076, US Patent No. 8,846,306).

A fundamental problem with all hemoglobin-based blood substitutes for transfusion is that they have very short residence times in the bloodstream. Thus, by way of example, the oxygen carrier of marine origin called HEMOXYCarrier®, developed by the company Hemarina on the basis of extracellular hemoglobin from the polychaete *Arenicola marina,* remains only a couple of days in circulation, disappearing completely from the system at 96 hours (Le Gall et al., 2014, In vivo biodistribution and oxygenation potential of a new generation of oxygen carrier. Journal of biotechnology, 187, 1-9). In this publication it was also determined that in half of the extracellular hemoglobin of *Arenicola marina* administered, fluorescence is lost at 24 hours (T½ = 24 h). Therefore, for this type of product to allow the treatment of a patient or an animal with acute anaemia, it would be required the transfusion of large amounts of hemoglobin-based blood substitutes and with a frequency not exceeding 2 days, at the most, so as to maintain its oxygen carrying capacity in the blood. This results in such products having limited potential applicability in the treatment of acute anaemia. For this reason, it is desirable to obtain a high-molecular-weight extracellular hemoglobin that is capable of stimulating erythropoiesis in the body to be treated. This would avoid having to supply high amounts of the product and, above all, it would eliminate the need to administer doses very frequently.

The present invention provides a stable, high-molecular-weight, extracellular hemoglobin, purified from an annelid native to Chile, preferably of the genus *Abarenicola* and more preferably of the species *Abarenicola pusilla* or *Abarenicola affinis,* which, in addition to its natural oxygen carrying capacity, allows to achieve, surprisingly, the induction of erythropoiesis in mammals. The latter is of great importance for the regeneration of red blood cells. In this way, a longer lasting effect is achieved in the treatment of anaemia, compared to that of blood substitutes of the state of the art.

With the extracellular hemoglobin purified from an annelid native to Chile, preferably of the genus *Abarenicola* and more preferably of the species *Abarenicola pusilla* or *Abarenicola affinis,* the surprising technical effect of controlling infections of bacterial origin is additionally achieved. In this way, the capacity of the extracellular hemoglobin of the present invention to activate erythropoiesis in mammals, simultaneously with the bactericidal activity, makes it a better product than other hemoglobins, for multiple special applications. A hemoglobin with both effects is particularly useful for critically ill patients in an intensive treatment unit, affected by acute anaemia and with high risks of hospital infection, for the seriously injured in remote or faraway places and with evident risks of microbial contamination, etc. In veterinary medicine it is very useful for treating domestic animals injured in fights, due to bleeding and the high possibility of bacterial infection. It is also important to consider, by way of example, that hemorrhagic septicemia is an acute and highly fatal form of pasteurellosis (caused by *Pasteurella multocida* serotypes 6:B and 6:E), which mainly affects cattle.

*In vitro* and *in vivo* transfusion tests of a composition comprising this hemoglobin have shown that it is not toxic, that it does not exhibit important side effects compatible with life, presenting characteristics suitable for its therapeutic use.

DETECTED DESCRIPTION OF THE INVENTION

The main object of the present invention is the use of the extracellular hemoglobin isolated and purified from an annelid native to Chile, preferably from the genus *Abarenicola* and more preferably from the species *Abarenicola pusilla* or *Abarenicola affinis,* for preparing a drug useful as a blood substitute to treat, at the same time, acute anaemia and bacterial infection in mammals.

In one embodiment of the invention, in the use of the isolated extracellular hemoglobin, an extracellular hemoglobin isolated from an annelid native to Chile is used, preferably from the genus *Abarenicola* and more preferably from the species *Abarenicola pusilla* or *Abarenicola affinis,* which allows the induction of the erythropoiesis and presents bactericidal activity, simultaneously.

In another embodiment of the invention, in the use of the isolated extracellular hemoglobin, an isolated extracellular hemoglobin which is a protein of molecular weight between 3000 and 5000 kDa is used.

In another embodiment of the invention, in the use of the isolated extracellular hemoglobin, an isolated extracellular hemoglobin having an absorption spectrum between 300-580 nm is used.

In another embodiment of the invention, in the use of the isolated extracellular hemoglobin, an isolated extracellular hemoglobin having a maximum absorbance between 400-420 nm and two absorbance peaks between 530-600 nm is used.

In another embodiment of the invention, in the use of the isolated extracellular hemoglobin, an isolated extracellular hemoglobin which is not toxic or has no side effects for mammals is used.

The second main object of the present invention is a pharmaceutical composition for the treatment of acute anaemia and bacterial infection in mammals, comprising an effective concentration of an extracellular hemoglobin isolated from an annelid native to Chile, preferably of the genus *Abarenicola* and more preferably of the species *Abarenicola pusilla* or *Abarenicola affinis,* and a pharmaceutically acceptable excipient.

In one embodiment of the invention, the composition comprises an isolated extracellular hemoglobin that allows the induction of erythropoiesis and exhibits bactericidal activity, simultaneously.

In another embodiment of the invention, the composition comprises an isolated extracellular hemoglobin which is a protein of molecular weight between 3000 and 5000 kDa.

In another embodiment of the invention, the composition comprises an isolated extracellular hemoglobin having an absorption spectrum between 300-560 nm.

In another embodiment of the invention, the composition comprises an isolated extracellular hemoglobin having an absorption spectrum between 300-560 nm.

In another further embodiment of the invention, the composition comprises an isolated extracellular hemoglobin having a maximum absorbance between 400-420 nm, and two absorbance peaks between 530-600 nm.

In one embodiment of the invention, the composition comprises an isolated extracellular hemoglobin at a concentration in the range of 0.5 - 4 g/L.

In another embodiment of the invention, the composition comprises an isolated extracellular hemoglobin at a concentration in the range of 1 - 3 g/L.

In another further embodiment of the invention, the composition comprises an isolated extracellular hemoglobin at a concentration in the range of 1.5 - 2.0 g/L.

In another preferred embodiment of the invention, the composition comprises an isolated extracellular hemoglobin at a concentration of 2.0 g/L.

In another embodiment of the invention, the composition comprises a pharmaceutically acceptable excipient which is a buffer solution comprising KCl, NaCl, $MgCl_2$, HEPES, NaOH.

In a further embodiment of the invention, the composition comprises KCl at the concentration range of 0 - 4 mM.

In a further embodiment of the invention, the composition comprises NaCl at the concentration range of 0 - 145 mM.

In a further embodiment of the invention, the composition comprises $MgCl_2$ at the concentration range of 0 - 0.2 mM.

In a further embodiment of the invention, the composition comprises HEPES at the concentration range of 0 - 10 mM.

In a further embodiment of the invention, the composition comprises NaOH at the concentration range of 0 - 0.1 M.

In a further embodiment of the invention, the composition is not toxic and has no side effects for mammals.

DESCRIPTION OF THE FIGURES

FIG. 1: This figure shows the isolation profile of *Abarenicola pusilla* hemoglobin by size exclusion chromatography from a diafiltered and ultra-concentrated sample.

FIG. 2: This figure is an example of comparison between the spectrophotometric profile of hemoglobin isolated from a hemolymph sample of *Abarenicola pusilla* (A) and hemoglobin isolated from a human blood sample (B).

FIG. 3: This figure shows an example of a spectrogram of the hemoglobin isolated from a hemolymph sample of *Abarenicola pusilla* at pH 5.0 measured at different times.

FIG. 4: This figure shows an example of a spectrogram of the hemoglobin isolated from a hemolymph sample of *Abarenicola pusilla* at pH 6.0 measured at different times.

FIG. 5: This figure shows example of a spectrogram of the hemoglobin isolated from a hemolymph sample of *Abarenicola pusilla* at pH 7.0 measured at different times.

FIG. 6: This figure shows an example of a spectrogram of the hemoglobin isolated from a hemolymph sample of *Abarenicola pusilla* at pH 8.0 measured at different times.

FIG. 7: This figure shows an example of a spectrogram of the hemoglobin isolated from a hemolymph sample of *Abarenicola pusilla* at pH 9.0 measured at different times.

FIG. 8: This figure shows an example of an antibiogram performed with the microorganism *Streptococcus agalactiae*, at different concentrations of hemoglobin isolated from *Abarenicola pusilla*, (1) saline solution only (negative control); (2) polychaete buffer only (negative control), and isolated hemoglobin from *Abarenicola pusilla* (3) 0.1 g/dL; (4) 0.5 g/dL; (5) 1.0 g/dL; (6) 1.5 g/dL; (7) as positive control.

FIG. 9: This figure shows a microscopic photograph of rodent reticulocytes transfused with polychaete hemoglobin at day 6. Image captured by optical microscopy, with 100X magnification.

FIG. 10: This figure is an example of a blood transfusion experiment with a hemoglobin composition isolated from *Abarenicola pusilla* in polychaete buffer, in twenty *Mus musculus* mice, Rockefeller strain. (X) represents the treatment with hemoglobin isolated from *Abarenicola pusilla* in polychaete buffer, (♦) represents the control treatment, only in polychaete buffer.

The following examples illustrate some specific applications of the invention, but are not intended to limit the description or scope of the present invention.

EXAMPLES

Example 1: Collection and conservation of hemoglobin samples obtained from *Abarenicola pusilla*.

The worms of the *Abarenicola pusilla* species, extracted from the coasts of southern Chile, are kept overnight in seawater at room temperature, in order to clean their digestive tubes of the sand content. Then, the hemolymph is extracted from the worm by puncturing the ventral vessel, using a 5 ml syringe with a 21 G needle. The contents of the syringe are poured into a funnel covered with glass wool, which captures the macroscopic particles present in the sample. The liquid content is recovered in an Erlenmeyer flask on ice to maintain the temperature of 4°C and thus avoid the dissociation of hemoglobin.

The contents of the flask are centrifuged for 15 minutes at 13,000g at 4°C to eliminate cellular debris contained in the sample. The supernatant containing the hemoglobin is collected and stored in a 2:1 ratio in a polychaete buffer solution (10 mM Hepes, 4 mM KCl, 145 mM NaCl, 0.2 mM $MgCl_2$, 7.0) adding 10 μl of Protease Inhibitor Cocktail Set III Calbiochem EDTA free, for each ml of hemoglobin dissolved in the buffer. Samples are stored at -20°C for later use and analysis.

Example 2: Purification of a hemoglobin from *Abarenicola pusilla*.

Protein purification was performed by taking a sample of the *Abarenicola pusilla* hemolymph, and by size exclusion chromatography. This technique allows the separation and recovery of proteins based on their molecular weight differences, where larger proteins are rapidly expelled with the mobile phase in polychaete buffer, while smaller proteins are retained inside the stationary phase, being slowly eliminated. Hemoglobin purification is performed by size exclusion chromatography, in a General Electric FPLC Akta Prime Plus equipment, using a GE Health care Hiprep TM16/60 Sephacryl Gel S-400 HR prepackaged column, whose protein separation range is from $2x10^3$ Da to $8x10^6$ Da. The column is equilibrated with 3 volumes of polychaete buffer and the same solution is used as elution buffer in the chromatography at a flow rate of 0.5 ml/min, using a sample volume of 2 ml at a concentration of 1 - 2 g/dl. At the end of the chromatography, the fractions that appear in the peak generated at 60 ml, corresponding to the purified hemoglobin, are collected. The purified hemoglobin fractions are stored at -20°C for later use.

With this method, 6 mL of purified hemoglobin with concentrations between 0.1 and 0.2 g/dL can be obtained. Subsequently, the hemoglobin is concentrated by ultrafiltration, using Amicon cells, preferably with stirring, reaching concentrations of 2.0 g/dL. Ultrafiltration consists of two stages; the first of diafiltration and the second of ultraconcentration, to this end the Amicon ultrafiltration system is used, which consists of three parts, which are: 10 ml capacity Millipore filtration cell model 8010 with 300 KDa cut off filter, 800 ml capacity Millipore RC800 Buffer Reservoir, and Millipore Selector Valve Model CDS-10.

Ultrafiltration membranes must be activated before use. For this, they are immersed in 0.1 M NaOH for 5 minutes. Then the ultrafiltration system is assembled, and the cell is loaded with 10 ml of sample, the process begins in liquid mode with diafiltration, at a pressure of 3 bars, with an efflux volume of 100 ml of polychaete buffer and at a temperature of 4°C. At this stage, the sample volume must be kept constant throughout the liquid filtration process.

Once the diafiltration is finished, the system is switched to gas mode and the ultraconcentration process is started at a pressure of 3 bars, at a temperature of 4°C. At this stage of the process, the sample is concentrated by reducing its volume to 2 ml. Finally, the samples obtained are stored at -20°C if they are to be used in a time greater than 8 hours, or kept at 4°C if they are to be used immediately.

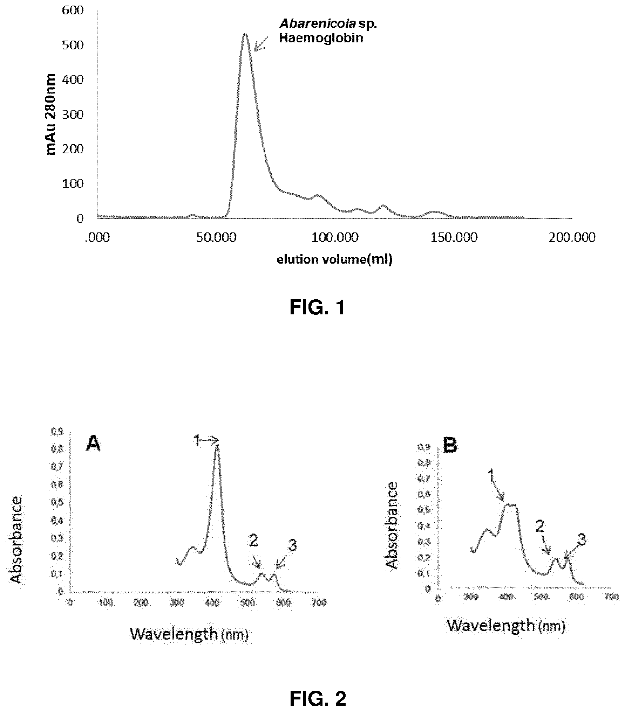

Fig. 1 shows the elution profile of a diafiltered and ultraconcentrated sample of hemolymph obtained from *Abarenicola pusilla*, with a volume of 180 mL of polychaete buffer, in which the purification of *Abarenicola pusilla* hemoglobin is observed at a 60 mL volume of elusion. The symmetry of the curve indicates that the hemoglobin was homogeneously purified.

The fraction obtained has a reddish color characteristic of the hemoglobin protein. The isolated extracellular hemoglobin described was characterized as having a molecular weight between 3000 - 5000 kDa. Due to the elution volume obtained, the approximate size of this protein is estimated to be 3000 - 4000 kDa.

Example 3: Determination of the absorption spectrum of *Abarenicola pusilla* hemoglobin.

The hemoglobin spectrogram is performed by measuring oxyhemoglobin based on the technique described by Bain et al., 2007 (Bain B. M Lewis Bates. Basic haematological techniques in Dacie and Lewis Practical Haematology, Madrid. Elsevier Spain S.A. 2007 pp. 24-27). In the determination, 50 $\mu$l of sample diluted in 950 ml of oxyhemoglobin reagent (0.4 gr/L ammonia solution) are used, homogenized by inversion, and immediately quantified using 1 ml of oxyhemoglobin reagent as blank. Using Genesus 10 UV Scanning spectrophotometer and quartz cuvettes, an absorbance scan of each sample is performed from 300 nm to 610 nm, at 5 nm intervals.

The presence of *Abarenicola pusilla* hemoglobin purified in FPLC is evaluated, performing a spectrogram of the samples contained in the collected hemoglobin fractions, in search of the Soret Band characteristic of hemoglobin molecules, at the same time the absorption spectrum obtained is compared against the spectrogram performed on a sample of human hemoglobin.

Fig. 2 shows the comparison of the spectrophotometric profiles of the extracellular hemoglobin isolated from *Abarenicola pusilla* hemolymph and the hemoglobin isolated from human blood. It can be observed that both profiles are similar, presenting a maximum peak (Soret band) at 414 nm, and two characteristic peaks (oxygenation peaks) at 540 nm and 575 nm. The maximum absorption presented by human hemoglobin is flattened, probably due to interference from cellular debris present in the sample. It was determined that the extracellular hemoglobin isolated from *Abarenicola pusilla* has a maximum absorption spectrum between 400 nm and 420 nm.

Example 4: Determination of the stability of *Abarenicola pusilla* hemoglobin at different pH values.

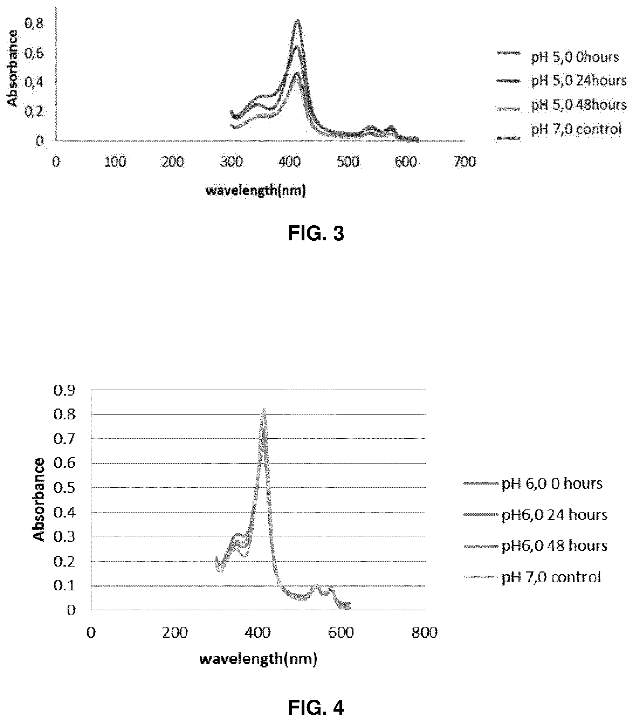

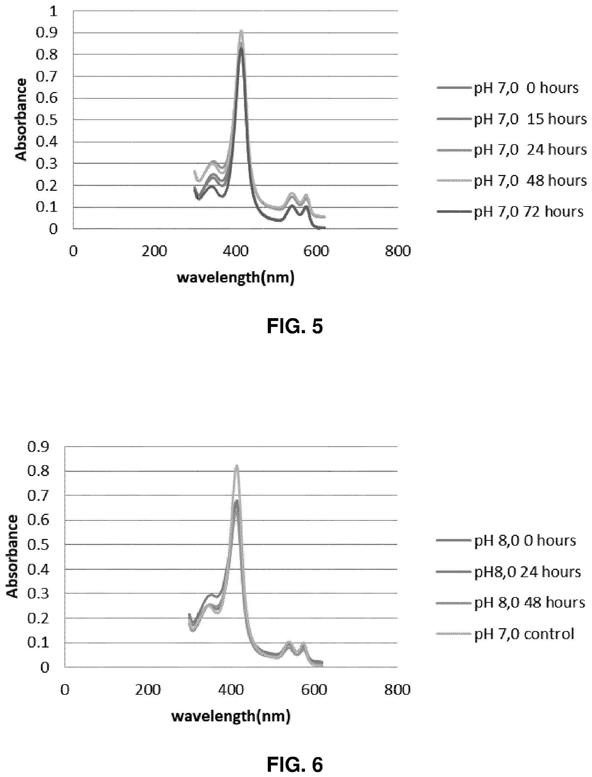

Spectrophotometric measurements of the protein were performed at different pHs, measured at 0, 24, and 48 hours, using the hemoglobin isolated from *Abarenicola pusilla* at pH 7.0 as a control. In Fig. 3, the analysis is shown at pH 5.0. In Fig. 4, this analysis is shown at pH 6.0. In Fig. 5, this analysis is shown at pH 7.0. In Fig. 6, this analysis is shown at pH 8.0. In Fig. 6, this analysis is shown at pH 9.0. In all the Figures, it can be observed that the protein does not precipitate and maintains its absorption spectrum between 300 and 560 nm. As demonstrated in the assays performed, the hemoglobin isolated from *Abarenicola pusilla* is a stable protein over time and does not precipitate at a pH between 5-9.

Example 5: Bactericidal activity of *Abarenicola pusilla* hemoglobin.

Another important characteristic of the invention is that the hemoglobin isolated from *Abarenicola pusilla* exhibits bactericidal activity.

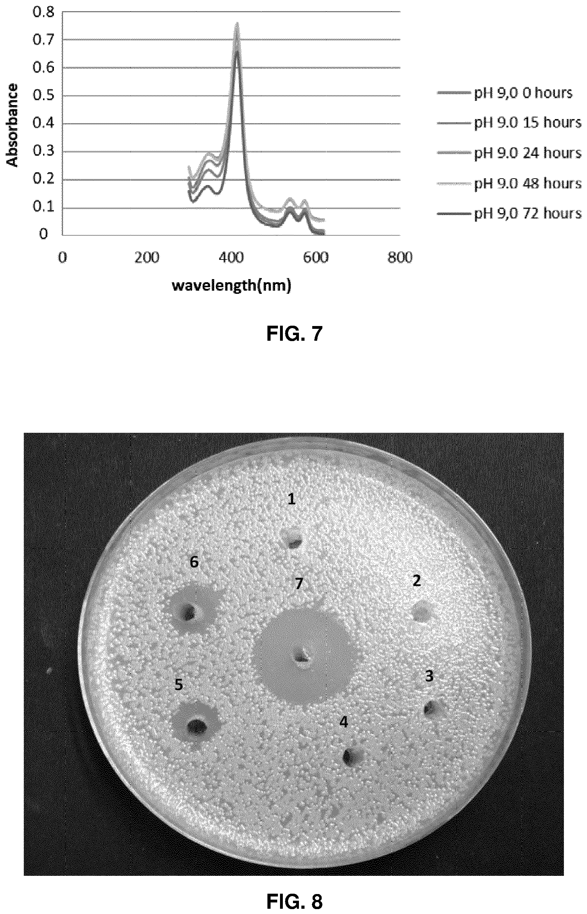

This activity is demonstrated by an antibiogram that was carried out on a lawn of bacteria of the *Streptococcus agalactiae* strain 27956 and samples of different concentrations of the hemoglobin isolated from *Abarenicola pusilla*. As a negative control, a polychaete buffer is used and it is observed that this solution does not generate an inhibition halo. The same occurs with hemoglobin isolated from *Abarenicola pusilla* at concentrations of 0.1 g/dL and 0.5 g/dL. However, at concentrations above 1 g/dL, a 7 mm inhibition halo is observed, and for concentrations of 1.5 g/dL, a 9 mm halo is observed. As a positive control, 1% penicillin/streptomycin is used, obtaining a halo of 15 mm. The results obtained are shown in Fig. 7.

Example 6: Effectiveness of *Abarenicola pusilla* hemoglobin in blood substitution.

To test the properties of hemoglobin, studies were conducted in mice of the Rockefeller strain, specifically 3-month-old females. These were anesthetized with a mixture of Ketamine/Pacifor: Physiological serum, intraperitoneally. Subsequently, 500 $\mu$L of blood was drawn from the retro-orbital plexus. After this, the "control group" was injected with a polychaete buffer via caudal vein. On the other hand, the "Hb group" was transfused extracellular hemoglobin isolated from *Abarenicola pusilla,* at a concentration of 0.8 g/dL, in the same volume as the blood previously drawn.

Among the variables studied are hematocrit and hemoglobin, controlled on day 1, 48 hours later, to verify that blood loss caused a decrease in hematological parameters, and on day 6, where it is already evident whether these are recovered. Finally, the study mice were euthanized.

Out of a total of 85 mice, the results of n=30 were analyzed. The other subjects were excluded, as their death was due to causes unrelated to the hemoglobin study (for example: anesthesia tests).

Of the 30 mice under study, 15 correspond to the group treated with polychaete buffer, (which was called "control group") and 15 to the group transfused with extracellular hemoglobin isolated from *Abarenicola pusilla,* which will be identified as "Hb group". Tables 1 and 2 show the results obtained in terms of survival and control of the variables hemoglobin and hematocrit.

Table 1: Results of the control group transfused with polychaete buffer.

| n= | Day 1 | | Day 3 | | Day 6 | |
|---|---|---|---|---|---|---|
| | Hematocrit (%) | Hemoglobin (g/dL) | Hematocrit (%) | Hemoglobin (g/dL) | Hematocrit (%) | Hemoglobin (g/dL) |
| 1 | 49 | 17,3 | 46 | 12,3 | | |
| 2 | 47 | 16,4 | 43 | 11,8 | 48 | 14 |
| 3 | 52 | 14,5 | | | | |
| 4 | 50 | 15,6 | 38 | 14,3 | 41 | 12,6 |
| 5 | 41 | 13,1 | | | | |
| 6 | 37 | 10,9 | | | | |
| 7 | 43 | 13,6 | | | | |
| 8 | 44 | 12,8 | | | | |
| 9 | 44 | 15,1 | 37 | 12,2 | | |
| 10 | 49 | 16,2 | 39 | 13,2 | 43 | 14,1 |
| 11 | 48 | 16,1 | 37 | 12,2 | | |
| 12 | 49 | 12,6 | | | | |
| 13 | 48 | 13,6 | | | | |
| 14 | 45 | 12,2 | - | | | |
| 15 | 47 | 15,4 | 36 | 13,1 | | |

† Blank spaces correspond to subjects who died during experimentation.

Table 2: Results of Hb group transfused with extracellular hemoglobin isolated from *Abarenicola pusilla.*

| n= | Day 1 | | Day 3 | | Day 6 | |
|---|---|---|---|---|---|---|
| | Hematocrit (%) | Hemoglobin (g/dL) | Hematocrit (%) | Hemoglobin (g/dL) | Hematocrit (%) | Hemoglobin (g/dL) |
| 1 | 46 | 15,9 | 44 | 11,5 | | |
| 2 | 45 | 15,3 | 39 | 15,2 | 45 | 14 |
| 3 | 45 | 15,9 | 35 | 15,4 | 43 | 13,5 |
| 4 | 45 | 14,7 | 38 | 14,9 | 47 | 12,3 |
| 5 | 45 | 17,3 | | | | |
| 6 | 50 | 15,1 | 40 | 12,5 | 44 | 14,2 |
| 7 | 46 | 14,3 | 41 | 12,3 | | |
| 8 | 49 | 14,1 | 38 | 12,4 | 46 | 13,8 |
| 9 | 48 | 13,4 | 35 | 11 | 46 | 14,2 |
| 10 | 45 | 12,8 | 40 | 12,1 | 45 | 15,1 |
| 11 | 46 | 15,3 | 38 | 13,1 | 46 | 15,2 |
| 12 | 48 | 16,2 | 39 | 13,2 | 47 | 16,3 |
| 13 | 49 | 16,1 | 40 | 12,9 | 48 | 16,1 |
| 14 | 46 | 15,4 | 35 | 12,3 | 45 | 15,1 |
| 15 | 45 | 14,9 | 38 | 13,1 | 47 | 15,9 |

† Blank spaces correspond to subjects who died during experimentation.

Rodents transfused with extracellular hemoglobin isolated from *Abarenicola pusilla* had a higher survival percentage and faster recovery of their hematological parameters, compared to mice treated only with polychaete buffer. This demonstrates the effectiveness of the extracellular hemoglobin isolated from *Abarenicola pusilla* in blood replacement, for the treatment of acute anaemia due to blood loss. The long duration of the protective effect of the extracellular hemoglobin isolated from *Abarenicola pusilla* is surprising, considering that a single initial dose was administered, and that a circulating half-life of only 24 hours has been described for the extracellular hemoglobin from the polychaete *Arenicola marina* (Le Gall et al., 2014, In vivo biodistribution and oxygenation potential of a new generation of oxygen carrier. Journal of biotechnology, 187, 1-9).

Example 7: Induction of hematopoiesis in the presence of *Abarenicola pusilla* hemoglobin in blood substitution.

In the same experiment described in Example 6, the reticulocyte count was performed to demonstrate the activation of hematopoiesis in the rodents transfused with extracellular hemoglobin isolated from *Abarenicola pusilla*. It is previously known that the percentage of reticulocytes in peripheral blood constitutes an important reflection of erythropoietic activity (Lewis, S.; B. Bain; I. Bates. 2008. Basic haematological techniques. In: Dacie and Lewis Practical Haematology. Madrid, Elsevier. Pp 32-33.).

Brilliant cresyl blue stain was used as a vital stain to perform the manual reticulocyte count, which was mixed in equal amounts with the blood obtained. It was incubated for 5 minutes at 37°C, and then a smear was prepared (Garcia et al., 2015).

$$\text{Formula:} \quad \text{Reticulocytes \%} = \frac{\text{No. of Reticulocytes counted x 100}}{1000}$$

Reticulocyte counts were performed in the studied mouse population, both on day 1 and day 6 of the experiment. For the sample on day 1, a n = 21 of blood samples taken prior to blood extraction with subsequent transfusion of extracellular hemoglobin isolated from *Abarenicola pusilla* were analyzed. The average obtained was 5.6%, to which $\pm$ 2 standard deviations were calculated, in order to obtain a range of normality in our studied population from 0% to 12%.

Table 3 shows the values obtained on the 6th day post experimentation and transfusion with extracellular hemoglobin isolated from *Abarenicola pusilla,* in a sample of 5 subjects.

Table 3: Reticulocyte count in mice transfused with extracellular hemoglobin isolated from *Abarenicola pusilla.*

| n = | 1 | 2 | 3 | 4 | 5 |
|---|---|---|---|---|---|
| Reticulocytes (%) | 21,2 | 21,6 | 37,4 | 39,2 | 25,4 |

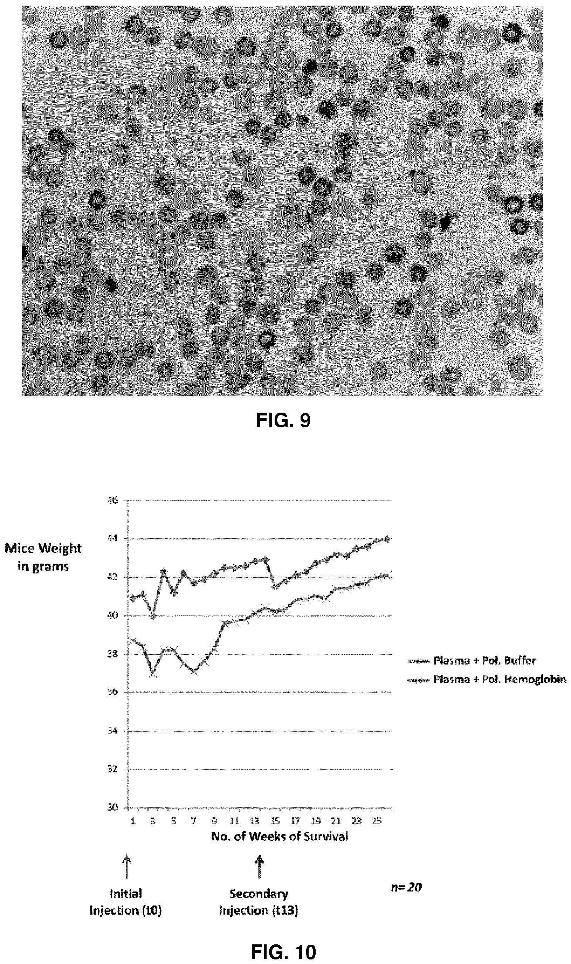

The results show that the mice reacted by increasing their percentage of reticulocytes. Fig. 9 shows an image obtained through optical microscopy, with 100X magnification. The result is a surprising technical effect, according to what is described in the state of the art. It is known that the lack of oxygen is the stimulus that produces an increase in the concentration of the glycoprotein hormone, erythropoietin, generated mainly in the kidneys. By means of erythropoietin, a signal is sent to the bone marrow that allows to activate an accelerated erythropoiesis, and to supply the body's requirement (Merino, M. 1998. Alteraciones de la eritropoyesis y del eritrocito. In: Fuentes, X.; M.J. Castiñeiras; J.M. Queralto. Bioquímica clínica y patología molecular. 2 ed. Barcelona, Reverté. Pp 1005-1006). However, in the presence of a transfused hemoglobin, a response from the bone marrow is not to be expected, since the transfused hemoglobin delivers oxygen to the body, making up for the deficit generated by acute blood loss. The foregoing shows that hematopoiesis activation occurs in rodents transfused with extracellular hemoglobin isolated from *Abarenicola pusilla.*

Example 8: Lack of toxic effects of a composition comprising hemoglobin isolated from *Abarenicola pusilla* in healthy mice.

The intravenous administration of the composition of hemoglobin isolated from *Abarenicola pusilla* is performed, undiluted or with a 50% dilution in plasma, in healthy mice. For this, this procedure was performed in 20 mice *(Mus musculus,* Rockefeller strain), to which 500 μL of blood was withdrawn by the jugular route under anesthesia, and 500 μL of the composition of hemoglobin isolated from of *Abarenicola pusilla* was administered at a concentration of 2 g/dL in polychaete buffer. All mice survived and lived for more than 5 months after transfusion (Fig. 10). This experiment indicates that hemoglobin isolated from *Abarenicola pusilla* is not toxic in healthy animals.

Example 9: Lack of toxic effects and unwanted side effects of a composition comprising hemoglobin isolated from *Abarenicola pusilla* in canines.

In another assay, the transfusion procedure was performed on a male canine, of approximately 3 years old, of mixed breed, which presented a fracture in the right hind limb in process of resolution, with normal vital signs. By analysis of the complete blood count and biochemical profile, the animal was healthy (Table 2). 5 ml of a composition of hemoglobin isolated from *Abarenicola pusilla* is administered intravenously at a concentration of 2 gr/dl in polychaete buffer.

Table 2: Biochemical and hematological examination pre injection (5 ml) of hemoglobin 2 gr/dl.

| Analysis | Sample Value | Reference Range | Mean Deviation |
|---|---|---|---|
| ALT | 58 U/L | 25 - 85 | 0,2 |
| AT | 33 U/L | 30 - 90 | -1,8 |
| Creatinine | 65 $\mu$mol/L | 35 - 115 | 0,5 |
| Urea | 5,2 $\mu$mol/L | 2,5 - 6,6 | 0,6 |

| Erythrogram | | |
|---|---|---|
| Analysis | Sample Value | Reference Range |
| Erythrocytes | 6,82 x 10^6 $\mu$L | 5,5-8,5 |
| Hematocrits | 45 % | 37-50 |
| Hemoglobin | 136 g/L | 120-180 |
| MCV | 66 fL | 60-77 |
| MCHC | 302 g/L | 320-370 |
| Nucleated Erythrocytes | 2 % | 0 |
| Anisocytosis | Rare | Rare |
| Polychromasia | Rare | Negative |
| Howell-Jilly | Negative | Negative |
| Platelets | 475000 $\mu$L | 200000-500000 |
| Proteins | 74 g/L | 55-75 |
| Fibrinogen | - g/L | <5 |

| Leukogram | | | | |
|---|---|---|---|---|
| | Relative Value (%) | | Absolute Range ($\mu$L) | |
| | Sample | Reference | Sample | Reference |
| Leukocytes | 100 | - | 18400 | 8000-14000 |
| Basophils | 0 | 0-1 | 0 | 0-200 |
| Eosinophils | 0 | 1-10 | 0 | 100-1500 |
| Neutrophils | 86 | 55-75 | 15824 | 3300-10000 |
| Band neutrophils | 1 | 0-3 | 184 | 0-300 |
| Juvenile neutrophils | 0 | 0 | 0 | 0 |
| Lymphocytes | 11 | 12-30 | 2024 | 1000-4500 |
| Monocytes | 2 | 1-7 | 368 | 100-700 |
| Not Classified | 0 | 0 | 0 | 0 |

Table 3 Biochemical and hematological examination post injection (5 ml) of hemoglobin 2 gr/dl.

| Analysis | Sample Value | Reference Range | Mean Deviation |
|---|---|---|---|
| ALT | 38 U/L | 25 - 85 | -1,1 |
| AT | 22 U/L | 30 - 90 | -2,5 |
| Creatinine | 49 $\mu$mol/L | 35 - 115 | -1,3 |
| Urea | 3,1 $\mu$mol/L | 2,5 - 6,6 | -1,5 |

(continued)

| Erythrogram | | |
|---|---|---|
| Analysis | Sample Value | Reference Range |
| Erythrocytes | 6,57 x 10^6 μL | 5,5-8,5 |
| Hematocrits | 43 % | 37-50 |
| Hemoglobin | 132 g/L | 120-180 |
| MCV | 65 fL | 60-77 |
| MCHC | 307 g/L | 320-370 |
| Nucleated Erythrocytes | 0% | 0 |
| Anisocytosis | Rare | Rare |
| Polychromasia | Negative | Negative |
| Howell-Jilly | Negative | Negative |
| Platelets | 483000 μL | 200000-500000 |
| Proteins | 78 g/L | 55-75 |
| Fibrinogen | - g/L | <5 |

| Leukogram | | | | |
|---|---|---|---|---|
| | Relative Value (%) | | Absolute Range (μL) | |
| | Sample | Reference | Sample | Reference |
| Leukocytes | 100 | - | 26400 | 8000-14000 |
| Basophils | 0 | 0-1 | 0 | 0-200 |
| Eosinophils | 1 | 1-10 | 264 | 100-1500 |
| Neutrophils | 81 | 55-75 | 21384 | 3300-10000 |
| Band neutrophils | 5 | 0-3 | 1320 | 0-300 |
| Juvenile neutrophils | 0 | 0 | 0 | 0 |
| Lymphocytes | 2 | 12-30 | 528 | 1000-4500 |
| Monocytes | 11 | 1-7 | 2904 | 100-700 |
| Not Classified | 0 | 0 | 0 | 0 |

During this procedure, the vital signs of the animal are constantly evaluated, without observing variations. It is kept under observation for the subsequent 5 days after administration, in which there is no clinical alteration. Complete blood count and biochemical profile analysis 4 days after administration show that there is no alteration in the parameters studied (Table 3). This experiment indicates that hemoglobin isolated from *Abarenicola pusilla* is not toxic and does not generate unwanted side effects in animals.

**Claims**

1. Use of the extracellular hemoglobin isolated from *Abarenicola pusilla,* **CHARACTERIZED in that** it is used for preparing a drug useful as a blood substitute to treat, at the same time, acute anaemia and bacterial diseases due to the bactericidal effect of said hemoglobin, in mammals.

2. Use of the isolated extracellular hemoglobin according to claim 1, **CHARACTERIZED in that** it is used, simultaneously, for formulating a drug useful to treat acute anaemia by inducing erythropoiesis and to treat bacterial infections.

3. A pharmaceutical composition for the treatment of acute anaemia and bacterial infection due to the bactericidal effect of this hemoglobin, in mammals, **CHARACTERIZED in that** it comprises an extracellular hemoglobin isolated from *Abarenicola pusilla* and a pharmaceutically acceptable excipient.

4. A composition according to claim 3, **CHARACTERIZED in that** said isolated extracellular hemoglobin allows the induction of erythropoiesis and presents bactericidal activity, simultaneously.

5. A composition according to claims 3 - 4, **CHARACTERIZED in that** said isolated extracellular hemoglobin is a protein having a molecular weight between 3000 and 5000 kDa.

6. A composition according to claims 3 - 5, **CHARACTERIZED in that** said isolated extracellular hemoglobin has an absorption spectrum between 300-580 nm.

7. A composition according to claims 3 - 5, **CHARACTERIZED in that** said isolated extracellular hemoglobin has a maximum absorbance between 400 - 420 nm, and two absorbance peaks between 530 - 600 nm.

8. A composition according to claims 3 - 7, **CHARACTERIZED in that** said composition comprises isolated extracellular hemoglobin at a concentration in the range of 0.5 - 4 g/L.

9. A composition according to claims 3 - 7, **CHARACTERIZED in that** said composition comprises isolated extracellular hemoglobin at a concentration in the range of 1 - 3 g/L.

10. A composition according to claims 3 - 7, **CHARACTERIZED in that** said composition comprises isolated extracellular hemoglobin at a concentration in the range of 1.5 - 2.0 g/L.

11. A composition according to claims 3 - 7, **CHARACTERIZED in that** the concentration of said isolated extracellular hemoglobin is 2.0 g/L.

12. A composition according to claims 3 - 11, **CHARACTERIZED in that** said pharmaceutically acceptable excipient is a buffer solution comprising KCl, NaCl, $MgCl_2$, HEPES, NaOH.

13. A composition according to claim 12, **CHARACTERIZED in that** said pharmaceutically acceptable excipient comprises KCl at the concentration range of 0 - 4 mM.

14. A composition according to claim 12, **CHARACTERIZED in that** said pharmaceutically acceptable excipient comprises NaCl at the concentration range of 0 - 145 mM.

15. A composition according to claim 12, **CHARACTERIZED in that** said pharmaceutically acceptable excipient comprises $MgCl_2$ at the concentration range of 0 - 0.2 mM.

16. A composition according to claim 12, **CHARACTERIZED in that** said pharmaceutically acceptable excipient comprises HEPES at the concentration range of 0 - 10 mM.

17. A composition according to claim 12, **CHARACTERIZED in that** said pharmaceutically acceptable excipient comprises NaOH at the concentration range of 0 - 0.1 M.

**FIG. 1**

**FIG. 2**

FIG. 3

FIG. 4

EP 3 842 062 A1

FIG. 5

FIG. 6

**FIG. 7**

**FIG. 8**

**FIG. 9**

Mice Weight
in grams

Plasma + Pol. Buffer
Plasma + Pol. Hemoglobin

No. of Weeks of Survival

Initial
Injection (t0)

Secondary
Injection (t13)

n= 20

**FIG. 10**

## INTERNATIONAL SEARCH REPORT

| International application No. |
| --- |
| PCT/CL2019/050073 |

**A.  CLASSIFICATION OF SUBJECT MATTER**

(CIP): A61K38/42, C07K14/805 (2019.01)

According to International Patent Classification (IPC) or to both national classification and IPC

**B.  FIELDS SEARCHED**

Minimum documentation searched (classification system followed by classification symbols)

(CIP): A61K38/42, C07K14/805

Documentation searched other than minimum documentation to the extent that such documents are included in the fields searched

Electronic data base consulted during the international search (name of data base and, where practicable, search terms used)

GOOGLE SCHOLAR, GOOGLE PATENTS, INAPI, STN

**C.  DOCUMENTS CONSIDERED TO BE RELEVANT**

| Category* | Citation of document, with indication, where appropriate, of the relevant passages | Relevant to claim No. |
| --- | --- | --- |
| Y | LE GALL, T. et al. In vivo biodistribution and oxygenation potential of a new generation of oxygen carrier. Journal of biotechnology, 2014, vol. 187, p. 1-9. DOI: 10.1016/j.jbiotec.2014.07.008 The whole document "Cited in the application". | 1-17 |
| Y | VIZIOLI, J. & SALZET, M. Antimicrobial peptides from animals: focus on invertebrates. Trends in pharmacological sciences, 2002, vol. 23, no 11, p. 494-496. DOI: 10.1016/S0165-6147(02)02105-3 The whole document | 1-17 |
| A | US 2003/0181358 A1 (Zal, F.) 25-09-2003 The whole document "Cited in the application". | |

| [X] Further documents are listed in the continuation of Box C. | [X] See patent family annex. |
| --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 29 November 2019 (29.11.2019) | 13 December 2019 (13.12.2019) |

| Name and mailing address of the ISA/ INAPI, Av. Libertador Bernardo O'Higgins 194, Piso 17, Santiago, Chile | Authorized officer NARVAEZ HURTADO, Claudio |
| --- | --- |
| Facsimile No. | Telephone No. |

Form PCT/ISA/210 (second sheet) (January 2015)

**INTERNATIONAL SEARCH REPORT**

| International application No. |
| --- |
| PCT/CL2019/050073 |

C (Continuation).    DOCUMENTS CONSIDERED TO BE RELEVANT

| Category* | Citation of document, with indication, where appropriate, of the relevant passages | Relevant to claim No. |
| --- | --- | --- |
| A | ROUSSELOT, M. et al. Arenicola marina extracellular hemoglobin: a new promising blood substitute. Biotechnology Journal: Healthcare Nutrition Technology, 2006, vol. 1, no 3, p. 333-345. DOI: 10.1002/biot.200500049 The whole document | |
| A | ZEBE, E. & SCHIEDEK, D. The lugworm Arenicola marina: a model of physiological adaptation to life in intertidal sediments. Helgoländer Meeresuntersuchungen, 1996, vol. 50, no 1, p. 37.[en línea], [recuperado el 2019-11-29]. Recuperado de Internet <https://hmr.biomedcentral.com/track/pdf/10.1007/BF02 367136> The whole document | |
| A | ZAL, F. & ROUSSELOT, M. Extracellular hemoglobins from annelids, and their potential use in biotechnology. Outstanding Marine Molecules, 2014, p. 361-376. DOI:10.1002/9783527681501.ch16 The whole document | |

Form PCT/ISA/210 (continuation of second sheet) (January 2015)

**INTERNATIONAL SEARCH REPORT**
Information on patent family members

International application No.

PCT/CL2019/050073

| US 2003/0181358 A1 | 25-09-2003 | US2008305178 (A1) | 11-12-2008 |
|---|---|---|---|
| | | WO0192320 (A2) | 06-12-2001 |
| | | WO0192320 (A3) | 04-04-2002 |
| | | AT272071 (T) | 15-08-2004 |
| | | AU6243601 (A) | 11-12-2001 |
| | | DE60104542 (T2) | 01-09-2005 |
| | | DK1284994 (T3) | 29-11-2004 |
| | | EP1284994 (A2) | 26-02-2003 |
| | | EP1284994 (B1) | 28-07-2004 |
| | | FR2809624 (A1) | 07-12-2001 |
| | | FR2809624 (B1) | 02-08-2002 |
| | | JP2004501118 (A) | 15-01-2004 |
| | | JP5001500 (B2) | 15-08-2012 |

Form PCT/ISA/210 (patent family annex) (January 2015)

## REFERENCES CITED IN THE DESCRIPTION

### Patent documents cited in the description

- US 7776526 B
- US 110936
- US 296982
- US 20120040453 A
- US 7999076 B
- US 8846306 B

### Non-patent literature cited in the description

- **HARDISON R.** A brief history of hemoglobins: Plant, animal, protist, and bacteria. *Proc. Natl. Acad. Sci USA,* 1996, vol. 93, 5675-79
- **CASTRO C. ; BRICENO JC.** *Perfluorocarbon-Based Oxygen Carriers: Review of Products and Trials Artif Organs,* 2010, vol. 34, 622-634
- **SHOEMAKER SA ; GERBER MJ ; EVANS GL ; ARCHER-PAIK LE ; SCOGGIN CH.** Initial clinical experience with a rationally designed, genetically engineered recombinant human hemoglobin. *Artif Cells Blood Substit Immobil Biotechnol,* 1994, vol. 22, 457-465
- **WEISKOPF R ; SILVERMAN T.** *Balancing potential risks and benefits of hemoglobin-based oxygen carriers Transfusion,* 2013, vol. 53, 2327-2333
- **GARLICK R. L. ; TERWILLIGER R. C.** Structure and oxygen equilibrium of hemoglobin and myoglobin from the pacific lugworm Abarenicola pacifica. *Comp. Biochem. Physiol.,* 1977, vol. 57B, 177-184
- **ROUSSELOT M ; DELPY E ; DRIEU LA RO-CHELLE C ; LAGENTE V ; PIROW R ; REES JF ; HAGEGE A ; LE GUEN D ; HOURDEZ S ; ZAL F.** Arenicola marina extracellular hemoglobin: a new promising blood substitute. *Biotechnol J.,* 2006, vol. 1, 333-345
- **LE GALL et al.** In vivo biodistribution and oxygenation potential of a new generation of oxygen carrier. *Journal of biotechnology,* 2014, vol. 187, 1-9
- Basic haematological techniques. **BAIN B ; M LEWIS BATES.** Practical Haematology. Elsevier, 2007, 24-27
- Basic haematological techniques. **LEWIS, S. ; B. BAIN ; I. BATES.** Practical Haematology. Elsevier, 2008, 32-33
- Alteraciones de la eritropoyesis y del eritrocito. **ME-RINO, M.** Bioquímica clínica y patología molecular. 1998, 1005-1006